# EUROPEAN PATENT APPLICATION

(11) **EP 3 647 787 A2**
(43) Date of publication of application: **06.05.2020**
(21) Application number: 18824813.2
(22) Date of filing: 02.07.2018
(51) Int. Cl.: G01N 33/569, G01N 33/543, G01N 33/53, G01N 21/65

(54) **DIAGNOSTIC KIT FOR SEPSIS AND DIAGNOSIS METHOD USING SAME**

(30) Priority: 30.06.2017 KR 20170083717
(71) Applicant: Korea Research Institute of Chemical Technology, Yuseong-gu, Daejeon 34114 (KR)
(72) Inventor: SUH, Yung Doug, Seoul 08016 (KR)
(74) Representative: Ström & Gulliksson AB
(86) International application number: PCT/KR2018/007487
(87) International publication number: WO 2019/004804

(57) **Abstract**

The present invention relates to a diagnostic kit for sepsis, comprising: a first core gold nanoparticle having a target capture oligonucleotide coupled thereto, the target capture oligonucleotide binding complementarily to a portion of a sepsis pathogen-specific genome; and a second core gold nanoparticle to which a target capture oligonucleotide having a Raman-active molecule coupled to one end thereof is coupled via the other end thereof, the target capture oligonucleotide including a sequence complementary to a portion of the sepsis pathogen-specific genome which does not overlap with, but is successive to the portion for the first gold nanoparticle, and a method for diagnosis of sepsis, using the same.

<110>     KOREA RESEARCH INSTITUTE OF CHEMICAL TECHNOLOGY

<120>     Kit for diagnosing sepsis and method for diagnosing sepsis using
          the same

<130>     OPA18216-PCT

<150>     10-2017-0083717
<151>     2017-06-30

<160>     59

<170>     KoPatentIn 3.0

<210>     1
<211>     40
<212>     DNA
<213>     Artificial Sequence

<220>
<223>     EC_target_SEQ1


<400>     1
gccgccttgc caggcattga cttcgacatc atcgtaatat                              40


<210>     2
<211>     20
<212>     DNA
<213>     Artificial Sequence

<220>
<223>     EC_target_capturing_probeA1


<400>     2
tcaatgcctg gcaaggcggc                                                    20


<210>     3
<211>     20
<212>     DNA
<213>     Artificial Sequence

<220>
<223>     EC_target_capturing_probeB1


<400>     3
atattacgat gatgtcgaag                                                    20


<210>     4
<211>     15
<212>     DNA
<213>     Artificial Sequence

<220>
<223>     protecting_probeA


<400>     4

gacctgaata accct                                                    15


<210>    5
<211>    15
<212>    DNA
<213>    Artificial Sequence

<220>
<223>    protecting_probeB


<400>    5
gatcgtcgtc taccg                                                    15


<210>    6
<211>    20
<212>    DNA
<213>    Artificial Sequence

<220>
<223>    EC_target_capturing_probeA2


<400>    6
ttttggcaac agggctaggt                                               20


<210>    7
<211>    21
<212>    DNA
<213>    Artificial Sequence

<220>
<223>    EC_target_capturing_probeB2


<400>    7
taatatcctg tcgaaaatcc t                                             21


<210>    8
<211>    20
<212>    DNA
<213>    Artificial Sequence

<220>
<223>    KP_target_capturing_probeA1


<400>    8
gggatatctg accagtcggg                                               20


<210>    9
<211>    20
<212>    DNA
<213>    Artificial Sequence

<220>
<223>    KP_target_capturing_probeB1

<400>    9
gaattaaaaa acaggaaatc                                                    20


<210>    10
<211>    20
<212>    DNA
<213>    Artificial Sequence

<220>
<223>    KP_target_capturing_probeA2


<400>    10
aggtcaacaa tgctgcggat                                                    20


<210>    11
<211>    20
<212>    DNA
<213>    Artificial Sequence

<220>
<223>    KP_target_capturing_probeB2


<400>    11
ctgcagaaac actgtacgtc                                                    20


<210>    12
<211>    20
<212>    DNA
<213>    Artificial Sequence

<220>
<223>    KP_target_capturing_probeA3


<400>    12
gtacttcgca aatctcaacg                                                    20


<210>    13
<211>    20
<212>    DNA
<213>    Artificial Sequence

<220>
<223>    KP_target_capturing_probeB3


<400>    13
caaatgaaca tcaaagcgaa                                                    20


<210>    14
<211>    20
<212>    DNA
<213>    Artificial Sequence

<220>

<223>    KP_target_capturing_probeA4

<400>    14
ttaactgccc tacactggtg                                                    20

<210>    15
<211>    20
<212>    DNA
<213>    Artificial Sequence

<220>
<223>    KP_target_capturing_probeB4

<400>    15
gacaatgtcg gtaaagttaa                                                    20

<210>    16
<211>    21
<212>    DNA
<213>    Artificial Sequence

<220>
<223>    KP_target_capturing_probeA5

<400>    16
gttaactgcc ctacactggt g                                                  21

<210>    17
<211>    21
<212>    DNA
<213>    Artificial Sequence

<220>
<223>    KP_target_capturing_probeB5

<400>    17
caaggtcttt tggggttatc g                                                  21

<210>    18
<211>    20
<212>    DNA
<213>    Artificial Sequence

<220>
<223>    KP_target_capturing_probeA6

<400>    18
ggtaccagag gtctcgtaaa                                                    20

<210>    19
<211>    20
<212>    DNA
<213>    Artificial Sequence

<220>
<223>    KP_target_capturing_probeB6


<400>    19
cagaggtctc gtaaaactga                                                    20


<210>    20
<211>    20
<212>    DNA
<213>    Artificial Sequence

<220>
<223>    SA_target_capturing_probeA1


<400>    20
tcacaaacag ataatggcgt                                                    20


<210>    21
<211>    20
<212>    DNA
<213>    Artificial Sequence

<220>
<223>    SA_target_capturing_probeB1


<400>    21
aaatagaagt ggttctgaag                                                    20


<210>    22
<211>    20
<212>    DNA
<213>    Artificial Sequence

<220>
<223>    SA_target_capturing_probeA2


<400>    22
ctatgattgt ggtagccatc                                                    20


<210>    23
<211>    20
<212>    DNA
<213>    Artificial Sequence

<220>
<223>    SA_target_capturing_probeB2


<400>    23
attattgtag gtgtattagc                                                    20


<210>    24
<211>    21

<212>    DNA
<213>    Artificial Sequence

<220>
<223>    SA_target_capturing_probeA3


<400>    24
cgaactaaag cttcgtttac c                                                21


<210>    25
<211>    20
<212>    DNA
<213>    Artificial Sequence

<220>
<223>    SA_target_capturing_probeB3


<400>    25
ccacgtccat atttatcagt                                                  20


<210>    26
<211>    20
<212>    DNA
<213>    Artificial Sequence

<220>
<223>    SA_target_capturing_probeA4


<400>    26
caatgttcta ccatagcgat                                                  20


<210>    27
<211>    20
<212>    DNA
<213>    Artificial Sequence

<220>
<223>    SA_target_capturing_probeB4


<400>    27
catacgatct ttacttatcc                                                  20


<210>    28
<211>    20
<212>    DNA
<213>    Artificial Sequence

<220>
<223>    EF_target_capturing_probeA1


<400>    28
gcttctttcc tcccgagtgc                                                  20

<210>    29
<211>    20
<212>    DNA
<213>    Artificial Sequence

<220>
<223>    EF_target_capturing_probeB1


<400>    29
ttgcactcaa ttggaaagag                                                    20


<210>    30
<211>    20
<212>    DNA
<213>    Artificial Sequence

<220>
<223>    EF_target_capturing_probeA2


<400>    30
gaagaacaag gacgttagta                                                    20


<210>    31
<211>    20
<212>    DNA
<213>    Artificial Sequence

<220>
<223>    EF_target_capturing_probeB2


<400>    31
actgaacgtc ccctgacggt                                                    20


<210>    32
<211>    20
<212>    DNA
<213>    Artificial Sequence

<220>
<223>    EF_target_capturing_probeA3


<400>    32
tggttaaact ttttgcctta                                                    20


<210>    33
<211>    20
<212>    DNA
<213>    Artificial Sequence

<220>
<223>    EF_target_capturing_probeB3


<400>    33
cgagcgaaga tcattggcac                                                    20

<210>    34
<211>    20
<212>    DNA
<213>    Artificial Sequence

<220>
<223>    EF_target_capturing_probeA4


<400>    34
ttttgaacat catcggtgac                                                    20


<210>    35
<211>    20
<212>    DNA
<213>    Artificial Sequence

<220>
<223>    EF_target_capturing_probeB4


<400>    35
catatagtct aagaaggctt                                                    20


<210>    36
<211>    20
<212>    DNA
<213>    Artificial Sequence

<220>
<223>    EF_target_capturing_probeA5


<400>    36
aatgtaataa ttggttcata                                                    20


<210>    37
<211>    20
<212>    DNA
<213>    Artificial Sequence

<220>
<223>    EF_target_capturing_probeB5


<400>    37
ccctgggtaa ataggtgctg                                                    20


<210>    38
<211>    20
<212>    DNA
<213>    Artificial Sequence

<220>
<223>    EF_target_capturing_probeA6

<400>    38
ggaatttcgt tcccgttact                                                    20


<210>    39
<211>    20
<212>    DNA
<213>    Artificial Sequence

<220>
<223>    EF_target_capturing_probeB6


<400>    39
ataccagttc gcaaaaagca                                                    20


<210>    40
<211>    20
<212>    DNA
<213>    Artificial Sequence

<220>
<223>    EF_target_capturing_probeA7


<400>    40
acgatccgaa aaccttcttc                                                    20


<210>    41
<211>    20
<212>    DNA
<213>    Artificial Sequence

<220>
<223>    EF_target_capturing_probeB7


<400>    41
gtccattgcc gaagattccc                                                    20


<210>    42
<211>    20
<212>    DNA
<213>    Artificial Sequence

<220>
<223>    EF_target_capturing_probeA8


<400>    42
ctttcgagcc tcagcgtcag                                                    20


<210>    43
<211>    20
<212>    DNA
<213>    Artificial Sequence

<220>
<223>    EF_target_capturing_probeB8

<400>    43
cagggtatct aatcctgttt                                                    20


<210>    44
<211>    20
<212>    DNA
<213>    Artificial Sequence

<220>
<223>    EF_target_capturing_probeA9


<400>    44
gtgtattagg tgaaggtgtt                                                    20


<210>    45
<211>    20
<212>    DNA
<213>    Artificial Sequence

<220>
<223>    EF_target_capturing_probeB9


<400>    45
tgatggccgt gtattaggtg                                                    20


<210>    46
<211>    20
<212>    DNA
<213>    Artificial Sequence

<220>
<223>    EF_target_capturing_probeA10


<400>    46
gaaatcgtcg taataaaccg                                                    20


<210>    47
<211>    20
<212>    DNA
<213>    Artificial Sequence

<220>
<223>    EF_target_capturing_probeB10


<400>    47
caagaaatcg tcgtaataaa                                                    20


<210>    48
<211>    20
<212>    DNA
<213>    Artificial Sequence

<220>
<223>    EF_target_capturing_probeA11


<400>    48
ggaattgaag gtattttaaa                                                    20


<210>    49
<211>    20
<212>    DNA
<213>    Artificial Sequence

<220>
<223>    EF_target_capturing_probeB11


<400>    49
attaacgcta ggagagccgg                                                    20


<210>    50
<211>    21
<212>    DNA
<213>    Artificial Sequence

<220>
<223>    EF_target_capturing_probeA12


<400>    50
cgactattta caagttcgcc c                                                  21


<210>    51
<211>    20
<212>    DNA
<213>    Artificial Sequence

<220>
<223>    EF_target_capturing_probeB12


<400>    51
atttacaagt tcgcccagag                                                    20


<210>    52
<211>    20
<212>    DNA
<213>    Artificial Sequence

<220>
<223>    PA_target_capturing_probeA1


<400>    52
cagcggtacg tccttgacca                                                    20


<210>    53
<211>    20
<212>    DNA

<213>    Artificial Sequence

<220>
<223>    PA_target_capturing_probeB1


<400>    53
gccagacatg tacgtcggcc                                                    20


<210>    54
<211>    20
<212>    DNA
<213>    Artificial Sequence

<220>
<223>    PA_target_capturing_probeA2


<400>    54
gtttccagtg cgtggtactg                                                    20


<210>    55
<211>    20
<212>    DNA
<213>    Artificial Sequence

<220>
<223>    PA_target_capturing_probeB2


<400>    55
gacgaagacg tacagcggcc                                                    20


<210>    56
<211>    20
<212>    DNA
<213>    Artificial Sequence

<220>
<223>    PA_target_capturing_probeA3


<400>    56
gcgctcagtc aggaaagcat                                                    20


<210>    57
<211>    20
<212>    DNA
<213>    Artificial Sequence

<220>
<223>    PA_target_capturing_probeB3


<400>    57
cgacaagaag gcgctcagtc                                                    20


<210>    58

<211>    20
<212>    DNA
<213>    Artificial Sequence

<220>
<223>    PA_target_capturing_probeA4


<400>    58
gacctggcag tgcattcttc                                                    20


<210>    59
<211>    20
<212>    DNA
<213>    Artificial Sequence

<220>
<223>    PA_target_capturing_probeB4


<400>    59
gtcattcgca gtggtccctg                                                    20

## Description

### [Technical Field]

The present invention relates to a diagnostic kit for sepsis, comprising a first core gold nanoparticle having an oligonucleotide coupled thereto, the oligonucleotide binding complementarily to a portion of a sepsis pathogen-specific genome, and a second core gold nanoparticle having an oligonucleotide coupled to the other end thereof having a Raman-active molecule coupled to one end, including a sequence complementary to a portion of the sepsis pathogen-specific genome which does not overlap with, but is successive to the portion for the first gold nanoparticle, and a method for diagnosis of sepsis, using the same.

### [Background Art]

Sepsis is an acute and severe disease in which a patient is infected by sepsis-causing pathogens such as bacteria, viruses, fungi, *etc*., and accordingly, the patient may suffer from high fever and eventually die. In this case, there are two main causes of death, and firstly, the immune system excessively functions due to so-called 'cytokine storm,' and such excessive autoimmune function destroys organs in the body (hyper-inflammation), and secondly, the propagation of the pathogens is not suppressed, causing the pathogens to spread to both blood and organs to paralyze all the functions of the organs (hypo-inflammation; immune paralysis).

Shock from sepsis is known as the main pathogenic mechanism mainly by systemic inflammatory reactions by various microorganisms, and this leads to arterial vasodilation and lowering of blood pressure that does not respond to blood pressure elevating agents. The mortality rate of septic shock patients is still high despite advances in diagnosis, monitoring, and treatment, and as we move into an aging society, the prevalence of septic shock continues to increase in older patients, which constitutes a big part of death in hospitals.

The incidence of sepsis in Korea is 347 per 100,000 people, and it is a fatal disease that is 3 times myocardial infarction and 1.5 times stroke, and the mortality rate is 31% (mortality rate of myocardial infarction and stroke is 9%). In particular, it is the cause of 60% of death for infants under 5 years old worldwide, and is the number one cause of death in 'the intensive care unit' (ICU) in Korea, and while the sepsis mortality rate is 20% in North America, Australia, and Europe, the sepsis mortality rate is 31%, which is somewhat high.

In this regard, the most important treatment for increasing the survival rate of sepsis patients is the rapid administration of appropriate antibiotics. The guidelines for the treatment of sepsis, published in 2012, recommend adequate administration of broad-spectrum antibiotics within three hours and blood culture tests to identify pathogens before the administration of antibiotics (Journal of the Korean Medical Association, 2012).

However, in clinical trials, results of blood culture tests can be diagnosed after at least 5 days in general and at least 24 hours after blood collection, and accordingly, most sepsis patients receive empirical antibiotics without identifying the exact pathogen. Therefore, if a bacterium that is resistant to empirical antibiotics such as multi-drug resistant bacteria is the pathogen for sepsis, when the results of the blood culture test confirm the infection with multi-drug resistant bacteria, there is a possibility that the treatment of the patient has already failed, leading to an increase in mortality rate.

Therefore, for faster diagnosis, a method of amplifying and detecting a gene through PCR may be considered, but due to the nature of PCR that amplifies even a small amount of genes, bacteria other than the pathogen of sepsis to be detected may be detected, or multiple bacteria may be detected, resulting in the misuse and/or abuse of antibiotics.

### [Disclosure]

### [Technical Problem]

As a result of research efforts to discover a kit and a method that can quickly and accurately diagnose sepsis as well as identify the type of pathogens without amplifying genes by PCR, the present inventors completed the present invention by confirming that by forming two core gold nanoparticle dimers in which oligonucleotides of complementary sequences are bound to a sepsis pathogen-specific genome, silver or gold shells are formed in controlled thickness, thereby controlling the distance between the particles within several nm, and by maximizing the surface enhanced Raman scattering effect, a Raman scattering signal can be checked to quickly and sensitively identify sepsis infection as well as the type of pathogen.

### [Technical Solution]

The first aspect of the present invention is to provide a diagnostic kit for sepsis, comprising a first core gold nanoparticle having a first target-capturing oligonucleotide coupled thereto via one end, the first target-capturing oligonucleotide binding complementarily to a portion of a first sepsis pathogen-specific genome; a second core gold nanoparticle having a second target-capturing oligonucleotide coupled thereto via one end, the second target-capturing oligonucleotide binding complementarily to the other portion of the first sepsis pathogen-specific genome; and a solution including a stabilizing agent, a reducing agent, and silver or gold ion, wherein any one of the first target-capturing oligonucleotide or the second target-capturing oligonucleotide has a first Raman-active molecule coupled to the other end thereof which does not bind to gold nanoparticles.

The second aspect of the present invention is to provide a method for providing information for diagnosis of sepsis, comprising a first step of preparing a specimen including a genome which is isolated from a sample collected from a subject suspected of having sepsis; a second step of reacting the specimen of the first step with a first core gold nanoparticle having a first target-capturing oligonucleotide coupled thereto via one end, the first target-capturing oligonucleotide binding complementarily to a portion of a sepsis pathogen-specific genome, and a second core gold nanoparticle having a second target-capturing oligonucleotide coupled thereto via one end, the second target-capturing oligonucleotide binding complementarily to the other portion of the sepsis pathogen-specific genome (wherein, any one of the first target-capturing oligonucleotide or the second target-capturing oligonucleotide has a Raman-active molecule coupled to the other end thereof which does not bind to gold nanoparticles); a third step of reacting the product of the second step with a solution including a stabilizing agent, a reducing agent, and silver or gold ion to simultaneously form a first shell and a second shell that are made of silver or gold on the first core gold nanoparticle and the second core gold nanoparticle, respectively; and a fourth step of measuring a Raman signal of a Raman-active molecule in the product obtained from the third step.

### [Advantageous Effect]

Since the kit of the present invention can form a structure showing a markedly increased Raman scattering signal when reacted with a sepsis pathogen-specific genome, even a small amount of samples can be quickly and accurately diagnosed in a few hours, leading to appropriate antibiotic administration, and thus, it can be widely used for lowering the morality rate through the early diagnosis of sepsis.

### [Brief Description of Drawings]

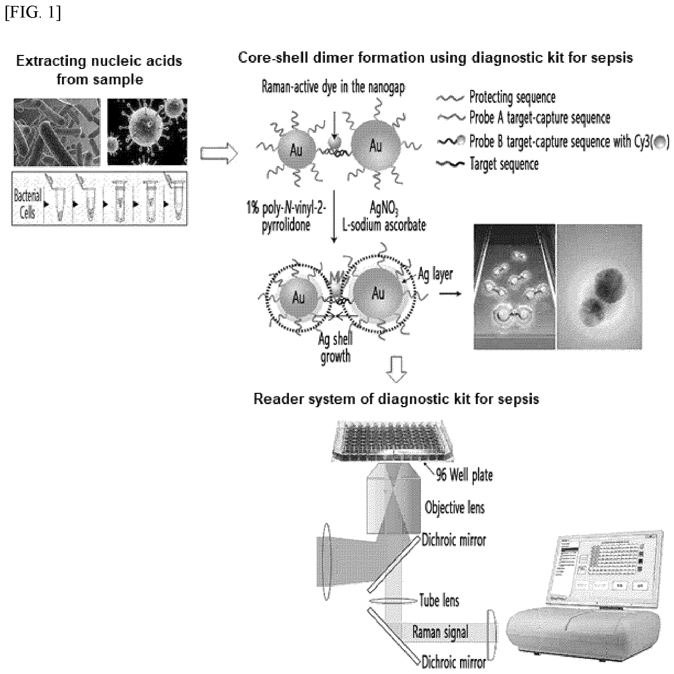

FIG. 1 is a diagram schematically showing the sepsis pathogen analysis method using the core-shell nanoparticle dimer formation by the diagnosis kit for sepsis according to the present invention.

### [Best Mode]

The first aspect of the present invention is to provide a diagnostic kit for sepsis, comprising a first core gold nanoparticle having a first target-capturing oligonucleotide coupled thereto via one end, the first target-capturing oligonucleotide binding complementarily to a portion of a first sepsis pathogen-specific genome; a second core gold nanoparticle having a second target-capturing oligonucleotide coupled thereto via one end, the second target-capturing oligonucleotide binding complementarily to the other portion of the first sepsis pathogen-specific genome; and a solution including a stabilizing agent, a reducing agent, and silver or gold ion, wherein any one of the first target-capturing oligonucleotide or the second target-capturing oligonucleotide has a first Raman-active molecule coupled to the other end thereof which does not bind to gold nanoparticles.

The second aspect of the present invention is to provide a method for providing information for diagnosis of sepsis, comprising a first step of preparing a specimen including a genome which is isolated from a sample collected from a subject suspected of having sepsis; a second step of reacting the specimen of the first step with a first core gold nanoparticle having a first target-capturing oligonucleotide coupled thereto via one end, the first target-capturing oligonucleotide binding complementarily to a portion of a sepsis pathogen-specific genome, and a second core gold nanoparticle having a second target-capturing oligonucleotide coupled thereto via one end, the second target-capturing oligonucleotide binding complementarily to the other portion of the sepsis pathogen-specific genome (wherein, any one of the first target-capturing oligonucleotide or the second target-capturing oligonucleotide has a Raman-active molecule coupled to the other end thereof which does not bind to gold nanoparticles); a third step of reacting the product of the second step with a solution including a stabilizing agent, a reducing agent, and silver or gold ion to simultaneously form a first shell and a second shell that are made of silver or gold on the first core gold nanoparticle and the second core gold nanoparticle, respectively; and a fourth step of measuring a Raman signal of a Raman-active molecule in the product obtained from the third step.

Hereinafter, the present invention will be described in detail.

The present invention is designed to provide a fast, accurate, and sensitive method for diagnosing sepsis and is based on ultra-sensitivity Raman spectroscopy using surface enhanced Raman scattering (SERS). Raman spectroscopy can obtain signals even for nonpolar molecules with a change in the induced polarization of molecules, and almost all organic molecules have their own Raman shift (cm⁻¹). In addition, it is also more suitable for the detection of biomolecules such as proteins, genes, *etc*., since it is not affected by the interference of water molecules. However, it was not commercialized due to low signal strength, but surface-enhanced Raman scattering is possible through various nanostructures, which enables signal detection at single-molecule levels. The SERS occurs when a Raman active molecule is adsorbed or located close to the metal surface, as it is a phenomenon in which a plasmon generated at the metal surface by laser that is projected for Raman measurement increases a signal emitted from Raman active molecules, SERS signal can be enhanced by hot spot, which is a strong electromagnetic field formed between dense nanoparticles. In this case, the SERS signal reacts sensitively to the size and shape of a nanoparticle, and/or the distance between the nanoparticle and a Raman-active material. However, as hot spot, which is formed by arbitrary agglomeration between nanoparticles, is unable to control these factors, it may impair the reproducibility of signal detection.

As such, the present inventors aim to provide a kit that can form a controllable nanostructure that can maximize the SERS effect to provide reproducible results so as to provide sensitive and reliable measurement results. Specifically, two core nanoparticles are spaced apart at regular intervals through DNA hybridization, and after forming a dimer including a Raman-active molecule therebetween, a gold or silver shell is formed at a controlled thickness at each core nanoparticle to be able to induce SERS enhancement by controlling the spacing of the two core-shell particles to the sub-nanometer level. Introduction of DNA to the surface of the core nanoparticle to form dimers by the hybridization thereof is a preferred means for maintaining a constant distance between nanoparticles. Double-stranded DNA formed by complementary binding has a constant structure and has a constant length of 0.34 nm per base pair, and thus the spacing between particles can be controlled by controlling the number of nucleotides constituting the same. Further, when additionally introducing DNA of any sequence to prevent nonspecific binding in addition to sequences designed for dimer formation on core nanoparticles, there is an advantage of blocking the formation of multimers and preparing dimers with high purity.

For the kit of the present invention, the term "first sepsis pathogen-specific genome" may refer to an oligonucleotide comprising a predetermined sequence capable of specifying the first pathogen through the presence of the genome. The genome may be the entire genome separated from the pathogen strain of sepsis, and may be a DNA fragment cut with a restriction enzyme to simplify the reaction, but is not limited thereto. In this case, for the restriction enzyme, HpaII(MspI), HaeIII, Hgal, or Taq(alpha)I may be used, but the restriction enzyme is not limited thereto. For example, a restriction enzyme capable of securing as many fragments as possible using a recognition sequence of 4 bp or less may be chosen and used. Specifically, Hpall or Taql may be used in consideration of the above conditions and economic efficiency. When designing the target oligonucleotide of the present invention and the corresponding target-capturing oligonucleotide, the above restriction enzymes, in particular, restriction sites of Hpall and Taql were applied.

For example, an oligonucleotide binding complementarily to a portion of the sepsis pathogen-specific genome may refer to an oligonucleotide that includes a portion of the sepsis pathogen-specific genome and a complementary sequence for hybridizing with the sepsis pathogen-specific genome.

As used herein, the term "target-capturing oligonucleotide" refers to an oligonucleotide capable of capturing a target oligonucleotide by hybridization because it has a sequence complementary to a portion of a gene to be detected, *i.e.,* a target oligonucleotide. In the present invention, the first target-capturing oligonucleotide and the second target-capturing nucleotide may each have a sequence that does not overlap with a sepsis pathogen-specific genome and is complementary to the other portion that is located consecutively or within several oligonucleotide gaps. Conversely, in the present invention, a target nucleotide may include a sequence complementary to the first target-capturing oligonucleotide and the second target-capturing nucleotide, and if present, the entire sequence including a gap of several oligonucleotides occurring therebetween.

The present invention is designed to prevent a high mortality rate due to delayed appropriate antibiotic treatment because a conventional blood culture test for diagnosing sepsis and identifying a pathogen takes at least 24 hours and several days. The present invention can provide a structure that can provide a markedly enhanced Raman signal by confirming the specific genome of sepsis pathogens and using a sequence complementary to the same, and is based on discovering that as a result, except that it takes several hours to pretreat a sample, it can measure and diagnose rapidly with high precision and sensitivity by Raman spectroscopy within 1 hour, and several pathogens can be identified simultaneously by simple measurement.

Meanwhile, as the Raman spectroscopic signal has a high spectral resolution due to its characteristics based on vibration spectroscopy, unlike fluorescent signals, the peak of the Raman signal is very sharp and there is little overlap of the spectrum, and thus, several pathogens can be diagnosed simultaneously by using a plurality of Raman active molecules showing the Raman signal at different wavelengths.

Specifically, the kit of the present invention may further comprise one or more pairs of a third core gold nanoparticle having a third target-capturing oligonucleotide coupled thereto via one end, the third target-capturing oligonucleotide binding complementarily to a portion of a second sepsis pathogen-specific genome, which is different from the first sepsis pathogen; and a fourth core gold nanoparticle having a fourth target-capturing oligonucleotide coupled thereto via one end, the fourth target-capturing oligonucleotide binding complementarily to the other portion of the second sepsis pathogen-specific genome such that multiple sepsis pathogens can be simultaneously detected, wherein any one of the third target-capturing oligonucleotide or the fourth target-capturing nucleotide has a second Raman-active molecule coupled to the other end thereof which do not bind to gold nanoparticles. Since the first target-capturing oligonucleotide, the second target-capturing oligonucleotide, the third target-capturing oligonucleotide, and the fourth target-capturing oligonucleotide are not in a competitive relationship with each other, they do not interfere with the interaction with each target and signals from each. One pair that is composed of a first core gold nanoparticle having a first target-capturing oligonucleotide coupled thereto via one end, the first target-capturing oligonucleotide binding complementarily to a portion of a first sepsis pathogen-specific genome; and a second core gold nanoparticle having a second target-capturing oligonucleotide coupled thereto via one end, the second target-capturing oligonucleotide binding complementarily to the other portion of the first sepsis pathogen-specific genome, and one pair that is composed of a third core gold nanoparticle having a third target-capturing oligonucleotide coupled thereto via one end, the third target-capturing oligonucleotide binding complementarily to a portion of a second sepsis pathogen-specific genome; and a fourth core gold nanoparticle having a fourth target-capturing oligonucleotide coupled thereto via one end, the fourth target-capturing oligonucleotide binding complementarily to the other portion of the second sepsis pathogen-specific genome, can be provided in the form of a mixture or each independently.

In this case, the third core gold nanoparticle and the fourth core gold nanoparticle may have the same size as any one and the other one among the first core gold nanoparticle and the second core gold nanoparticle, respectively, or are all independent. For example, while oligonucleotides may all be included in which two or more pairs of core gold nanoparticles may be included where nucleic acids having one sequence are bound to one core gold nanoparticle, or two or more types of sepsis pathogen-specific genome can be bound to one core gold nanoparticle, a core gold nanoparticle can be used that is designed to include a Raman-active molecule showing Raman signals at different wavelengths. These particles can be used to detect two or more types of sepsis pathogen at the same time.

The first core gold nanoparticle and the third core gold nanoparticle included in the kit of the present invention may each independently have an average diameter of 10 nm to 50 nm, and the second core gold nanoparticle and the fourth core gold nanoparticle may be selected in combination to have an average diameter of 1 to 2 times than that of the first core gold nanoparticle and the third core gold nanoparticle. In diagnosing sepsis using the kit of the present invention, the degree of enhancement of Raman signals that is finally measured may depend on the distance between the gold or silver shell formed on the core gold nanoparticle. However, the measured Raman signals are not independent of not only the distance between core-shell nanoparticle dimers that are finally formed, but also the size and the distance of each constituting core, that is, the distance of the target oligonucleotide, and these have a mutually organic relationship.

As described above, while one core gold nanoparticle has a size selected from a range of 10 nm to 50 nm, the other core gold nanoparticle which will form a dimer therewith may select particles having a size of 1 to 2 times, preferably 1.3 to 1.7 times the selected size. More preferably, particles having a size of 1.4 to 1.6 times may be selected, but are not limited thereto.

Further, the first target-capturing oligonucleotide and the second target-capturing oligonucleotide, and the third target-capturing oligonucleotide and the fourth target-capturing oligonucleotide have the same number of bases included therein or a difference within ±5 bases, and the total sum of the number of bases of the first target-capturing oligonucleotide and the second target-capturing oligonucleotide and the total sum of the number of bases of the third target-capturing oligonucleotide and the fourth target-capturing oligonucleotide may each independently be 15 to 100, preferably 25 to 70, and more preferably 35 to 60, but are not limited thereto.

For example, in order to provide the most efficiently enhanced Raman signal, considering the size of the core gold nanoparticles described above, it is preferable to select the distance of target-capturing oligonucleotide coupled to each core gold nanoparticle, that is, the first target-capturing oligonucleotide, the second target-capturing oligonucleotide, the third target-capturing oligonucleotide, and the fourth target-capturing oligonucleotide, so as to complementarily bind to the target oligonucleotide to be detected. Specifically, based on that when the size of the first core gold nanoparticle and the third core gold nanoparticle is 20 nm and, the total sum of the number of bases of the first target-capturing oligonucleotide and the second target-capturing oligonucleotide and the total sum of the number of base of the third target-capturing oligonucleotide and the fourth target-capturing oligonucleotide are 30, the size of the first core gold nanoparticle and the total sum of the number of bases of the first target-capturing oligonucleotide and the second target-capturing oligonucleotide, and the size of the third core gold nanoparticle and the total sum of the number of bases of the third target-capturing oligonucleotide and the fourth target-capturing oligonucleotide may be designed to be proportional. For example, while the total sum of the number of bases of the first target-capturing oligonucleotide and the second target-capturing oligonucleotide and the total sum of the third target-capturing oligonucleotide and the fourth target-capturing oligonucleotide are selected in the range described above, when the size of the first or third core gold nanoparticle that is used is 20 nm, it may be preferable to increase or decrease proportionally within 20% deviation based on the increase and decrease of the size based on the number of bases of 30.

In addition, the Raman active molecule in a complex formed when the target oligonucleotide is coupled by introducing a Raman-active molecule at the other end where the core gold nanoparticle of any one of the oligonucleotide among the first target-capturing oligonucleotide and the second target-capturing oligonucleotide is not bound, is located between the first core gold nanoparticle and the second core gold nanoparticle, and gold or silver shell in each core gold nanoparticle is formed at the same rate. In the finally formed core-shell nanoparticle dimer, it is preferable that the Raman active molecule is located close to the center of the distance between these particles in order to be located in a nanogap formed between these shells to show the maximum SERS effect. Therefore, it is preferable that the first target-capturing oligonucleotide and the second target-capturing oligonucleotide bound to each core are designed to have similar lengths to each other. For example, when the difference between the lengths of the first target-capturing oligonucleotide and the second target-capturing oligonucleotide is large, the Raman active molecule is embedded in either shell and may not display Raman signals when a shell is formed to have a nanogap of several nm.

In addition, when the size of the particles used is smaller than the above-mentioned range, the size of the Raman signal by itself may be small so that it may be difficult to obtain a desired level of sensitivity even when amplified by the SERS effect. Meanwhile, when greater than the above range, the unit dimer may be unnecessarily large, or the binding with the target oligonucleotide may be hindered by steric effect by its size. In addition, as described above, the distance between the core particles increases due to an increase in the number of target nucleotide bases according to the increase in the size of the core gold nanoparticles to obtain an efficient Raman signal, and thus, the shell particles need to be formed to be unnecessarily thick, and the waste of reaction time and/or reagents may be accompanied.

In a specific exemplary embodiment of the present invention, the sizes of the first core gold nanoparticle and the second core gold nanoparticle are chosen to be 40 nm and 60 nm, respectively, and accordingly, the first target-capturing oligonucleotide and the second target-capturing oligonucleotide were configured to include complementary sequences with their 20 consecutive sequences so as to complementarily bind to 40 bases of the target nucleotide.

The first sepsis pathogen and the second pathogen that can be diagnosed by using the kit of the present invention may be each independently selected from the group consisting of *Escherichia coli, Klepsiella pneumoniae, Staphylococcus aureus, Streptococcus pyogenes, Enterococcus faecalis,* and *Pseudomonas aeruginosa.* The above-listed strains are sepsis pathogens that are well known in the art, and the present invention is not limited to these strains, and may be applied without limitation to strains capable of causing sepsis.

While the kit of the present invention is designed to show an enhanced signal to detect a trace amount of a sepsis pathogen, the wrong diagnosis due to the nature of sepsis may lead to the misuse and abuse of drugs, and it is characterized in that each element is optimized so that more accurate diagnosis is possible.

In the kit of the present invention, the first core gold nanoparticle, the second core gold nanoparticle, the third core gold nanoparticle, and the fourth core gold nanoparticle may each independently be spherical particles having a diameter of 20 nm to 100 nm, but are not limited thereto. Specifically, the first core gold nanoparticles to the fourth core gold nanoparticles may be spherical having a circularity of 0.9 to 1.0 with a standard deviation of not more than ±0.05. Since commercially available gold nanoparticles have a polyhedron shape rather than a sphere, the basic structure of core gold nanoparticles is maintained when a silver or gold shell is introduced, and when a silver or gold shell is introduced with a uniform thickness, there may be a difference in the spacing between both core-shell nanoparticles in the dimer formed according to the microstructure of the oligonucleotide-linked portion. Therefore, in order to form a nanogap of more uniform size, it is preferable to use as spherical core gold particles as possible, thereby obtaining a more uniform and predictably controlled Raman signal. Specifically, since a dimer formed by the combination of complete spheres show point-to-point interactions, the amplification effect on signals from Raman-active molecules located between them can be rather low, but there is an advantage that a signal can be obtained that is controlled in a more accurate and desired and/or predictable direction without any further interference, *etc.* On the other hand, dimers formed in a heterogeneous form, that is, a combination of particles of polyhedrons close to a circle, are point-to-point, point-to-line, point-to-face, line-to-line, line-to face, or face-to-face selection and any uncontrollable interaction may occur. This can lead to higher signal enhancement effects due to the increase in contact area, but the uncertainty of the signals due to their heterogeneous mixture, that is, the band broadening of the spectrum is inevitable. Therefore, for more accurate diagnosis, it is preferable to use fully spherical core gold nanoparticles.

In addition, another factor that may obscure the signal being measured is to consider a case of forming a cluster rather than a dimer, that is, combining a plurality of the second core gold nanoparticles with one first core gold nanoparticle. The formation of clusters can lead to band broadening and/or peak position shifts of the measured Raman signal. Therefore, for the first core gold nanoparticle and the second core gold nanoparticle to combine at 1:1, respectively, a protecting oligonucleotide may be further introduced on the surface of each core gold nanoparticle in addition to the first target-capturing oligonucleotide and the second target-capturing oligonucleotide. The protecting oligonucleotide may be composed of any sequence that does not bind non-specifically to the target oligonucleotide or the genome that may exist in the strain to be detected. In this case, the amount of the protecting oligonucleotide used may be determined depending on the size of core gold nanoparticles to be applied. For example, based on a particle having a size of 15 nm, the target-capturing oligonucleotide (first to fourth target-capturing oligonucleotide) and the protecting oligonucleotide may be used at a ratio of 1:90 to 1:110 and may be used by increasing or decreasing proportionally depending on the increase or decrease of the size of a particle. In a specific exemplary embodiment of the present invention, the ratio of 1:99 was used for a particle having a diameter of 15 nm, and the ratio of 1:199 was used for a particle having a diameter of 30 nm.

The oligonucleotide included in the kit of the present invention is characterized by binding complementarily to a portion of a sepsis pathogen-specific genome. The oligonucleotide can be selected by the following procedure. For example, genome information of sepsis pathogens is obtained from a known database, and among them, sequences that are involved in intracellular metabolic circuits and are less likely to cross-react between strains are analyzed and selected, and oligonucleotide pairs of appropriate length and sequence can be designed based on the above. The oligonucleotide may be DNA or RNA, but in the case of RNA, since stability is not guaranteed, DNA may be preferably used. After selecting 10 to 15 candidate groups by the above method, a Raman complex using the kit of the present invention can be formed on the basis of the above, and it can be included in the kit by measuring the signal and selecting an oligonucleotide sequence having high molecular diagnostic effectiveness.

In the kit of the present invention, any one of the first target-capturing oligonucleotide and the second target-capturing oligonucleotide may bind to the first core gold nanoparticle and the second core gold nanoparticle, respectively, via the 5' end and the other via the 3' end, respectively, and any one of the third target-capturing oligonucleotide and the fourth target-capturing oligonucleotide may bind to the third core gold nanoparticle and the fourth core gold nanoparticle, respectively, via the 5' end and the other via the 3' end, respectively.

Specifically, the first target-capturing oligonucleotide and the second target-capturing oligonucleotide may all include 5 to 20 nucleotide sequences that are complementary to a portion of the first sepsis pathogen-specific genome, and the third target-capturing oligonucleotide and the fourth target-capturing oligonucleotide may all include 5 to 20 nucleotide sequences that are complementary to a portion of the second sepsis pathogen-specific genome, respectively. In this case, it is preferable that these sequences do not overlap with each other, and it is preferable that they are complementary to neighboring sequences spaced within several sequences from each other. For example, the first target-capturing oligonucleotide and the second target-capturing oligonucleotide, and the third target-capturing oligonucleotide and the fourth target-capturing oligonucleotide may be connected to each other, or may be located adjacent to each other within 3 nucleotide intervals.

For example, when the first sepsis pathogen-specific genome is composed of 30 amino acid sequences, in order to hybridize to 15 sequences from the 3' end to the 5' end of the first sepsis pathogen-specific genome, the first target-capturing oligonucleotide may be composed to include a sequence complementary thereto. Further, in order to hybridize 15 sequences from the 16^{th} sequence from the 3' end to the 5' end direction, that is, 15 sequences from the 5' end to the 3' end, the second target-capturing oligonucleotide may be composed to include a sequence complementary thereto. In this case, a thiol group may be attached to the 5' end of the first target-capturing oligonucleotide so as to bind to the first core gold nanoparticle directly thereby or via a linker. Meanwhile, the second target-capturing oligonucleotide may have a thiol group at the 3' end so as to bind to the first core gold nanoparticle directly thereby or via a linker. As such, the Raman active molecule may be coupled to the 3' end of the first target-capturing oligonucleotide or the 5' end of the second target-capturing oligonucleotide, and may be coupled thereto directly or via a linker composed of 1 to 3 sequences, but is not limited thereto.

Configuring as the above, when the first target-capturing oligonucleotide and the second target-capturing oligonucleotide include A₁₀-PEG₁₈ as a linker, a gold nanoparticle may obtain a bound dimer in which gold nanoparticles are separated by about 15 nm. When the reaction time and/or conditions are adjusted to form a silver or gold shell at a thickness of 5 nm, a gold core-silver or gold shell dimer having a nanogap of 5 nm may be obtained where a Raman-active molecule is located in the nanogap. The linker was introduced to allow a target-capturing oligonucleotide to be located in an upright form without lying on the surface of a core gold nanoparticle, and these linkers are attached to the surface and have little effect on the distance between core gold nanoparticles.

For example, the first target-capturing oligonucleotide and the second target-capturing oligonucleotide may all include 5 to 20 nucleotide sequences complementary to a portion of the first sepsis-specific genome, and the third target-capturing oligonucleotide and the fourth target-capturing oligonucleotide may all include 5 to 20 nucleotide sequences complementary to a portion of the second sepsis pathogen-specific genome, and these sequences do not overlap, wherein the first target-capturing oligonucleotide and the second target-capturing oligonucleotide, and the third target-capturing oligonucleotide and the fourth target-capturing oligonucleotide may be adjacently positioned at an interval of 1 to 3 nucleotides, respectively, but are not limited thereto.

The first or second Raman-active molecule may be an organic fluorescent molecule. For example, it may be selected from the group consisting of FAM(6-carboxyfluorescein; 6-FAM), Dabcyl(4-((4-(dimethylamino)phenyl)azo)benzoic acid or 4-((4-(dimethylamino)phenyl)azo)benzoate), tetrameethyl rhodamine isothiol (TRIT), NBD (7-nitrobenz-2-1,3-diazole), Texas red dye, phthalic acid, terephthalic acid, isophthalic acid, cresyl fast violet, cresyl Blue violet, brilliant cresyl blue, para-aminobenzoic acid, erythrosine, biotin, digoxigenin, 5-carboxy-4', 5-dichloro-2',7'-dimethoxy, fluorescein, 5-carboxy-2 ',4',5',7'-tetrachlorofluorescein, 5-carboxyfluorescein, 5-carboxy rhodamine, 6-carboxyrhodamine, 6-carboxytetramethyl aminophthalocyanine, azomethine, cyanine, xanthine, succinylfluorescein, aminoacrydine, quantum dot, carbon nanotube, carbon allotropes, cyanide, thiol, chlorine, bromine, methyl, phosphorus, sulfur, and cyanine dyes (Cy3, Cy3.5, and Cy5), and rhodamine. In this case, in order to facilitate simultaneous detection of multiple pathogens, the first or second Raman active molecule may be selected by combining different ones, for example those that have no overlapping of Raman spectra. Meanwhile, the organic fluorescent molecule may display a Raman signal as well as a fluorescent signal, and in order to minimize the covering by the fluorescent signal, a material having a low fluorescence yield may be selected, but the present invention is not limited thereto.

In the method for providing information for the diagnosis of sepsis of the present invention, when the Raman signal of the Raman active molecule measured from the fourth step is significantly increased compared to the Raman signal measured from the same Raman active molecule labeled on a gold-core-silver or gold-shell nanoparticle of the same size, the subject is judged to be infected by a sepsis pathogen which includes a sequence complementary to the first target-capturing oligonucleotide and the second target-capturing oligonucleotide in genome.

When the sepsis pathogen-specific genome included in a specimen, which is isolated from a sample collected from a subject, is hybridizable, including a sequence complementary to an oligonucleotide bound to the first core gold nanoparticle and the second core gold nanoparticle, the first core gold nanoparticle and the second core gold nanoparticle are linked via an oligonucleotide bound thereto and the sepsis pathogen-specific genome that is hybridized thereto to form a dumbbell-type dimer. In this case, any one of the first target-capturing oligonucleotide or the second target-capturing oligonucleotide is at the other end not bound to the gold nanoparticle, and the Raman-active molecule will be located in the middle of the core gold nanoparticle and the second gold nanoparticle. Therefore, in the case of forming a silver or gold shell in the formed dimer, the gap between the nanoparticles on both sides becomes narrower, and by controlling the thickness of a shell, the gap between nanoparticles can be controlled to have a narrow nanogap of 0.5 nm to 10 nm, specifically, 0.5 nm to 5 nm, and more specifically, 0.5 nm to 3 nm. The final structure thus formed has a nanogap of several nm, and particles of a gold core-silver or gold shell structure connected by complementary binding of oligonucleotides in which the Raman-active molecule is located in the nanogap are formed. Accordingly, the Raman-active molecule located in the nanogap between particles of a gold core-silver or gold shell structure generates a Raman signal when irradiated with light at an appropriate wavelength, and since the Raman signal generated at this time represents an augmented signal of about 10¹⁰ times or more, it is possible to detect a small amount of genome by Raman spectroscopy. Moreover, since the types of pathogens can be distinguished within a significantly faster time than the blood culture test, early diagnosis of sepsis and early prescription of appropriate antibiotics may be possible.

### [Detailed Description]

Hereinafter, preferred examples are provided to aid in understanding the present invention. However, the following examples are merely provided to more easily understand the present invention, and the contents of the present invention are not limited by the examples.

### Example 1. Preparation of gold core-silver shell nanoparticle with Raman-active molecule (Cy3) located at the junction of two nanoparticles

A Raman-active gold core-silver shell dimer was synthesized based on the DNA-directed bridging method of silver shell formation controlled by the amount of gold particles to which a target nucleotide is coupled and additionally by the amount of a silver precursor.

First, by a precise control and effective purification step of the molar ration of protecting and target-capturing oligonucleotide sequences, highly purified gold nanoparticle dimer structures with target oligonucleotides were obtained. Specifically, a target nucleotide including 40 bases represented by SEQ ID NO. 1, which is a portion of the genome of *Escherichia coli,* was selected (5'-GCCGCCTTGCCAGGCATTGACTTCGACATCATCGTAATAT-3'; Tₘ = 67.6°C), and in order to detect this, a target-capturing oligonucleotide was designed to include base sequences each represented by SEQ ID NOs.2 and 3 (SEQ ID NO. 2; Probe A; 5'-TCAATGCCTGGCAAGGCGGC/iSp9/A30/SH-3'; Tₘ = 64.2°C and SEQ ID NO. 3; Probe B; 5'-HS/A30/iSp9/ATATTACGATGATGTCGAAGAAA/Raman-active molecule/-3'; Tₘ = 54.2°C). A protecting oligonucleotide was composed of any sequence to block binding to the target nucleotide (SEQ ID NO. 4; Protecting A; 5'-GACCTGAATAACCCT/iSp9/A10/SH-3'; Tₘ = 50.4°C and SEQ ID NO. 5; Protecting B; 5'-HS/A10/iSp9/GATCGTCGTCTACCG-3'; Tₘ = 61.0°C).

Since the maximum gap distance (d; gap distance) between the surfaces of a gold nanoparticle (AuNP) and a Raman-active molecule still reached 7 nm, it was necessary to reduce the spacing to obtain amplified electromagnetic gain. Meanwhile, in the SERS detection, since silver has an effect several times better than gold, silver nano-shell was introduced to control the distance between the shell surface and the Raman-active molecule. In this case, for the Raman-active molecule, various organic fluorescent molecules having different fluorescence wavelengths and/or intensities, such as Cy3, Dabcyl, FAM, *etc.* were substituted and tested. As a result, when Dabcyl was introduced which has somewhat weaker fluorescence than Cy3 with high fluorescence intensity, it was confirmed that it was more advantageous for Raman signal detection by reducing the hiding of the Raman signal by fluorescence.

Specifically, to reduce the gap distance between nanoparticles to make amplified SERS signal, the thickness of a silver nanoshell was adjusted to nanometer level by a known modification method (Chem. Comm. 2008, J. Phys. Chem. B 2004, 108, 5882-5888), and the surface of the gold nanoparticle dimer was coated. A gold nanoparticle dimer solution 250 µM was reacted with various amounts of AgNO₃ (10⁻³ M), under the presence of 100 µL of poly(vinyl) pyrrolidone as a stabilizing agent and 50 µL of L-sodium ascorbic acid (10⁻¹ M) as a reducing agent in a 0.3 M PBS solution, for 3 hours at room temperature. Gold core-silver shell heterodimeric nanoparticles with silver shell thicknesses of about 3 nm, 5 nm, and 10 nm were synthesized using 30 µL, 40 µL, and 70 µL of a AgNO₃ solution (10⁻³ M), respectively. By this method, the silver shell thickness was successfully controlled in nano units such as about 3 nm, 5 nm, 10 nm, *etc*., thereby obtaining a gold-silver core-shell heterodimer structure coupled with a target oligonucleotide.

By conventional synthetic methods, to control one target-capturing oligonucleotide sequence to be modified on the surface of a gold nanoparticle, gold nanoparticles (20 nm) for probe A were functionalized with 3 ' terminal thiol-modified oligonucleotide sequences of two types, probe A and protection A. Gold nanoparticles (40 nm) for probe B were also functionalized by the 5' end-thiol-modified oligonucleotide sequences of two types, probe B and protection B. For the molar ratio of the two types of sequences, based on the nanoparticle-size dependent loading ability of oligonucleotides on the surface of gold nanoparticles, the ratio of protecting oligonucleotide: target-capturing oligonucleotide was controlled to be 132:1 for probe A, and 266:1 for probe B. Specifically, experiments were performed by controlling the amount of a protecting oligonucleotide used so that the gold nanoparticle of probe A and the nanoparticle of probe B could form a 1:1 bonded dimer rather than a cluster. By setting the ratio of a protecting oligonucleotide: target-capturing oligonucleotide to 99:1 for particles having a diameter of 15 nm, and adjusting proportionally according to the size of particles, it was confirmed that it was possible to block multimer formation and a dimer could be prepared. Importantly, for the target-capturing oligonucleotide sequence for probe B, a Raman active molecule Cy3, which functions as a Raman tag, was coupled to the end. Oligonucleotide-modified probes A and B were also purified by a magnetic separation technique to remove monomer particles to which the target-capturing oligonucleotide was not bound. The tosyl group of magnetic beads (1 µm in diameter, Invitrogen) was substituted and combined by the amine-modified oligonucleotide complementary sequence to the target-capturing sequences of probe A or B, respectively. Only gold nanoparticles bound to the target-capturing sequence could be separated by magnetic beads. Next, the purified probes A and B solutions were hybridized with a sufficient amount of target oligonucleotide sequences in 0.3 M PBS.

### Example 2: Improved accuracy depending on the shape of core particles

In order to improve the accuracy of measurement, two fully spherical gold nanoparticles whose size was controlled were selected as a core. Synthesis was performed using the method proposed by Prof. Gira Yi of Sungkyunkwan University (ACS Nano, 2013, 7(12): 11064-11070*).* Specifically, an octahedron having a larger size than the spherical particles to be prepared was synthesized as an initial particle and etched to prepare completely spherical gold nanoparticles having a circularity of 0.94 or more. As particles are agglomerated by cetyl trimethylammonium bromide (CTAB) that is used for synthesis or the introduction of DNA via a thiol group on the surface of particles is disadvantageous, the surface was modified to have a negative zeta potential by treatment with a surfactant such as Tween 20, *etc.*

### Example 3: Selection of target-capturing oligonucleotide for diagnosis of sepsis

A target oligonucleotide for the diagnosis of sepsis was selected from the genome of *Escherichia coli, Klepsiella pneumoniae, Staphylococcus aureus, Enterococcus faecalis,* and *Pseudomonas aeruginosa* that are the representative strains known to cause sepsis. Two to twelve target-capturing oligonucleotide pairs were selected to specifically bind to a target oligonucleotide including at least two types of 40 to 115 bases each selected from the genome of the five strains. Specifically, in addition to the olignonucleotides represented by SEQ ID NOs: 2 and 3 used in Example 1 for *E. coli,* a sequence pair represented by SEQ ID NOs: 6 and 7 (SEQ ID NO. 6: 5'-TTTTGGCAACAGGGCTAGGT-3' and SEQ ID NO. 7: 5'-TAATATCCTGTCGAAAATCCT-3') were additionally tested.

Further, a series of target-capturing oligonucleotide pairs represented by SEQ ID NOs: 8 to 19 (total of 6 pairs), 20 to 27 (total of 4 pairs), 28 to 51 (total of 12 pairs), and 52 to 59 (total of 4 pairs) that were designed for the detection of the genome of *Klepsiella pneumonia* (KP), *Staphylococcus aureus* (SA), *Enterococcus faecalis* (EF), and *Pseudomonas aeruginosa* (PA) were used for testing, and each sequence is described below.
SEQ ID NO: 8; 5'-GGGATATCTGACCAGTCGGG-3'
SEQ ID NO: 9; 5'-GAATTAAAAAACAGGAAATC-3'
SEQ ID NO: 10; 5'-AGGTCAACAATGCTGCGGAT-3'
SEQ ID NO: 11; 5'-CTGCAGAAACACTGTACGTC-3'
SEQ ID NO: 12; 5'-GTACTTCGCAAATCTCAACG-3'
SEQ ID NO: 13; 5'-CAAATGAACATCAAAGCGAA-3'
SEQ ID NO: 14; 5'-TTAACTGCCCTACACTGGTG-3'
SEQ ID NO: 15; 5'-GACAATGTCGGTAAAGTTAA-3'
SEQ ID NO: 16; 5'-GTTAACTGCCCTACACTGGTG-3'
SEQ ID NO: 17; 5'-CAAGGTCTTTTGGGGTTATCG-3'
SEQ ID NO: 18; 5'-GGTACCAGAGGTCTCGTAAA-3'
SEQ ID NO: 19; 5'-CAGAGGTCTCGTAAAACTGA-3'
SEQ ID NO: 20; 5'-TCACAAACAGATAATGGCGT-3'
SEQ ID NO: 21; 5'-AAATAGAAGTGGTTCTGAAG-3'
SEQ ID NO: 22; 5'-CTATGATTGTGGTAGCCATC-3'
SEQ ID NO: 23; 5'-ATTATTGTAGGTGTATTAGC-3'
SEQ ID NO: 24; 5'-CGAACTAAAGCTTCGTTTACC-3'
SEQ ID NO: 25; 5'-CCACGTCCATATTTATCAGT-3'
SEQ ID NO: 26; 5'-CAATGTTCTACCATAGCGAT-3'
SEQ ID NO: 27; 5'-CATACGATCTTTACTTATCC-3'
SEQ ID NO: 28; 5'-GCTTCTTTCCTCCCGAGTGC-3'
SEQ ID NO: 29; 5'-TTGCACTCAATTGGAAAGAG-3'
SEQ ID NO: 30; 5'-GAAGAACAAGGACGTTAGTA-3'
SEQ ID NO: 31; 5'-ACTGAACGTCCCCTGACGGT-3'
SEQ ID NO: 32; 5'-TGGTTAAACTTTTTGCCTTA-3'
SEQ ID NO: 33; 5'-CGAGCGAAGATCATTGGCAC-3'
SEQ ID NO: 34; 5'-TTTTGAACATCATCGGTGAC-3'
SEQ ID NO: 35; 5'-CATATAGTCTAAGAAGGCTT-3'
SEQ ID NO: 36; 5'-AATGTAATAATTGGTTCATA-3'
SEQ ID NO: 37; 5'-CCCTGGGTAAATAGGTGCTG-3'
SEQ ID NO: 38; 5'-GGAATTTCGTTCCCGTTACT-3'
SEQ ID NO: 39; 5'-ATACCAGTTCGCAAAAAGCA-3'
SEQ ID NO: 40; 5'-ACGATCCGAAAACCTTCTTC-3'
SEQ ID NO: 41; 5'-GTCCATTGCCGAAGATTCCC-3'
SEQ ID NO: 42; 5'-CTTTCGAGCCTCAGCGTCAG-3'
SEQ ID NO: 43; 5'-CAGGGTATCTAATCCTGTTT-3'
SEQ ID NO: 44; 5'-GTGTATTAGGtGAAGGTGTT-3'
SEQ ID NO: 45; 5'-TGATGGCCGTGTATTAGGTG-3'
SEQ ID NO: 46; 5'-GAAATCGTCGTAATAAACCG-3'
SEQ ID NO: 47; 5'-CAAGAAATCGTCGTAATAAA-3'
SEQ ID NO: 48; 5'-GGAATTGAAGGTATTTTAAA-3'
SEQ ID NO: 49; 5'-ATTAACGCTAGGAGAGCCGG-3'
SEQ ID NO: 50; 5'-CGACTATTTACAAGTTCGCCC-3'
SEQ ID NO: 51; 5'-ATTTACAAGTTCGCCCAGAG-3'
SEQ ID NO: 52; 5'-CAGCGGTACGTCCTTGACCA-3'
SEQ ID NO: 53; 5'-GCCAGACATGTACGTCGGCC-3'
SEQ ID NO: 54; 5'-GTTTCCAGTGCGTGGTACTG-3'
SEQ ID NO: 55; 5'-GACGAAGACGTACAGCGGCC-3'
SEQ ID NO: 56; 5'-GCGCTCAGTCAGGAAAGCAT-3'
SEQ ID NO: 57; 5'-CGACAAGAAGGCGCTCAGTC-3'
SEQ ID NO: 58; 5'-GACCTGGCAGTGCATTCTTC-3'
SEQ ID NO: 59; 5'-GTCATTCGCAGTGGTCCCTG-3'

After culturing each strain, the suitability of diagnosing sepsis through the detection of each strain using the target-capturing oligonucleotide pairs designed above was confirmed, including a series of procedures for separating and amplifying genomic DNA (gDNA) by PCR.

Further, representatively, the suitability of the target oligonucleotide for the confirmation of gDNA in sepsis patients by *E. coli* was confirmed again. First, the sensitivity of the target oligonucleotide was confirmed by a specimen in which *E. coli* was detected through a blood culture test. In addition, by using a specimen of a sepsis patient in which *E*. *coli* was not confirmed by the blood culture test, but *E. coli* infection was confirmed by histological examination, the sensitivity of the target oligonucleotide was confirmed.

## Claims

1. A diagnostic kit for sepsis, comprising:
a first core gold nanoparticle having a first target-capturing oligonucleotide coupled thereto via one end, the first target-capturing oligonucleotide binding complementarily to a portion of a first sepsis pathogen-specific genome;
a second core gold nanoparticle having a second target-capturing oligonucleotide coupled thereto via one end, the second target-capturing oligonucleotide binding complementarily to the other portion of the first sepsis pathogen-specific genome; and
a solution comprising a stabilizing agent, a reducing agent, and silver or gold ion,
wherein any one of the first target-capturing oligonucleotide or the second target-capturing oligonucleotide has a first Raman-active molecule coupled to the other end thereof which does not bind to gold nanoparticles.

2. The diagnostic kit for sepsis of claim 1, further comprising:
one or more pairs of a third core gold nanoparticle having a third target-capturing oligonucleotide coupled thereto via one end, the third target-capturing oligonucleotide binding complementarily to a portion of a second sepsis pathogen-specific genome, which is different from the first sepsis pathogen; and a fourth core gold nanoparticle having a fourth target-capturing oligonucleotide coupled thereto via one end, the fourth target-capturing oligonucleotide binding complementarily to the other portion of the second sepsis pathogen-specific genome such that multiple sepsis pathogens can be simultaneously detected,
wherein any one of the third target-capturing oligonucleotide or the fourth target-capturing nucleotide has a second Raman-active molecule coupled to the other end thereof which does not bind to gold nanoparticles.

3. The diagnostic kit for sepsis of claim 2, wherein the third core gold nanoparticle and the fourth core gold nanoparticle have the same size as any one of and the other one of the first core gold nanoparticle and the second core gold nanoparticle, respectively, or are each independent.

4. The diagnostic kit for sepsis of claim 1 or 2, wherein the first core gold nanoparticle and the third core gold nanoparticle each independently have an average diameter of 10 nm to 50 nm, and the second core gold nanoparticle and the fourth core gold nanoparticle have an average diameter that is 1 to 2 times than that of the first core gold nanoparticle and the third core gold nanoparticle, respectively.

5. The diagnostic kit for sepsis of claim 1 or 2, wherein the first target-capturing oligonucleotide and the second target-capturing oligonucleotide, and the third target-capturing oligonucleotide and the fourth target-capturing oligonucleotide have the same number of bases comprised therein or a difference within ±5, and the total sum of the number of bases of the first target-capturing oligonucleotide and the second target-capturing oligonucleotide, and the total sum of the number of bases of the third target-capturing oligonucleotide and the fourth target-capturing oligonucleotide are each independently 15 to 100.

6. The diagnostic kit for sepsis of claim 1 or 2, wherein, based on that when the first core gold nanoparticle and the third core gold nanoparticle have a size of 20 nm and, the total sum of the number of bases of the first target-capturing oligonucleotide and the second target-capturing oligonucleotide, and the total sum of the number of bases of the third target-capturing oligonucleotide and the fourth target-capturing oligonucleotide are 30, the size of the first core gold nanoparticle and the total sum of the number of bases of the first target-capturing oligonucleotide and the second target-capturing oligonucleotide and the size of the third core gold nanoparticle and the total sum of the number of bases of the third target-capturing oligonucleotide and the fourth target-capturing oligonucleotide are proportional thereto, respectively.

7. The diagnostic kit for sepsis of claim 1 or 2, wherein the first core gold nanoparticle to the fourth core gold nanoparticle are in a spherical form having a circularity of 0.9 to 1.0 with a standard deviation of not more than ±0.05.

8. The diagnostic kit for sepsis of claim 1 or 2, wherein the first sepsis pathogen and the second sepsis pathogen are each independently selected from the group consisting of *Escherichia coli, Klepsiella pneumoniae, Staphylococcus aureus, Streptococcus pyogenes, Enterococcus faecalis,* and *Pseudomonas aeruginosa.*

9. The diagnostic kit for sepsis of claim 1 or 2, wherein the first core gold nanoparticle, the second core nanoparticle, the third core gold nanoparticle, and the fourth core gold nanoparticle are complete spherical particles each independently having a diameter of 20 nm to 100 nm.

10. The diagnostic kit for sepsis of claim 1 or 2, wherein any one of the first target-capturing oligonucleotide and the second target-capturing oligonucleotide binds to the first core gold nanoparticle and the second core gold nanoparticle, respectively, via the 5' end and the other via the 3' end, respectively, and any one of the third target-capturing oligonucleotide and the fourth target-capturing oligonucleotide binds to the third core gold nanoparticle and the fourth core gold nanoparticle, respectively, via the 5' end and the other via the 3' end, respectively.

11. The diagnostic kit for sepsis of claim 1 or 2, wherein the first target-capturing oligonucleotide and the second target-capturing oligonucleotide all comprise 5 to 20 nucleotide sequences complementary to a portion of the first sepsis pathogen-specific genome, and the third target-capturing oligonucleotide and the fourth target-capturing oligonucleotide all comprise 5 to 20 nucleotide sequences complementary to a portion of the second sepsis pathogen-specific genome, and these sequences do not overlap, wherein the first target-capturing oligonucleotide and the second target-capturing oligonucleotide, and the third target-capturing oligonucleotide and the fourth target-capturing oligonucleotide are adjacently positioned at an interval of 0 to 3 nucleotides, respectively.

12. The diagnostic kit for sepsis of claim 1 or 2, wherein the first or second Raman-active molecule is an organic fluorescent molecule showing a Raman signal at different wavelengths.

13. The diagnostic kit for sepsis of claim 1 or 2, wherein the first Raman-active molecule and the second Raman-active molecule show a low fluorescence signal.

14. A method for providing information for diagnosis of sepsis, comprising:
a first step of preparing a specimen comprising a genome which is isolated from a sample collected from a subject suspected of having sepsis;
a second step of reacting the specimen of the first step with a first core gold nanoparticle having a first target-capturing oligonucleotide coupled thereto via one end, the first target-capturing oligonucleotide binding complementarily to a portion of a sepsis pathogen-specific genome, and a second core gold nanoparticle having a second target-capturing oligonucleotide coupled thereto via one end, the second target-capturing oligonucleotide binding complementarily to the other portion of the sepsis pathogen-specific genome (wherein, any one of the first target-capturing oligonucleotide or the second target-capturing oligonucleotide has a Raman-active molecule coupled to the other end thereof which does not bind to gold nanoparticles);
a third step of reacting the product of the second step with a solution comprising a stabilizing agent, a reducing agent, and silver or gold ion to simultaneously form a first shell and a second shell that are made of silver or gold on the first core gold nanoparticle and the second core gold nanoparticle, respectively; and
a fourth step of measuring a Raman signal of a Raman-active molecule in the product obtained from the third step.

15. The method of claim 14, wherein the sepsis pathogen is selected from the group consisting of *Escherichia coli, Klepsiella pneumoniae, Staphylococcus aureus, Streptococcus pyogenes, Enterococcus faecalis,* and *Pseudomonas aeruginosa.*

16. The method of claim 14, wherein, when the Raman signal of the Raman active molecule measured from the fourth step is significantly increased compared to the Raman signal measured from the same Raman active molecule labeled on a gold-core-silver or gold-shell nanoparticle of the same size, the subject is judged to be infected by a sepsis pathogen which comprises a sequence complementary to the first target-capturing oligonucleotide and the second target-capturing oligonucleotide in genome.

17. The method of claim 14, wherein the third step is performed until the shortest distance between the first shell and the second shell is 0.5 nm to 10 nm.
